# EUROPEAN PATENT APPLICATION

(11) **EP 4 035 654 A1**
(43) Date of publication of application: **03.08.2022**
(21) Application number: 21165160.9
(22) Date of filing: 26.03.2021
(51) Int. Cl.: A61K 9/00, A61K 31/135, A61P 25/16, A61K 9/20, A61K 47/38, A61K 47/32, A61K 47/26

(54) **AN ORODISPERSIBLE PHARMACEUTICAL SOLID DOSAGE FORM OF RASAGILINE**

(30) Priority: 30.01.2021 IN 202121004135
(71) Applicant: Intas Pharmaceuticals Limited, Ahmedabad - 380054, Gujrat (IN)
(72) Inventor: LLUCH LORES, María Carmen, 08039 Barcelona (ES); FORMOSA MÁRQUEZ, Xavier, 08192 Sant Quirze del Vallès (ES); DOMÍNGUEZ FERNÁNDEZ, Almudena, 31110 Noáin (ES); TORREA GOÑI, Diego, 31110 Noáin (ES)
(74) Representative: Ter Meer Steinmeister & Partner

(57) **Abstract**

The present invention relates to an orodispersible pharmaceutical solid dosage form comprising a therapeutically effective amount of rasagiline or a pharmaceutically acceptable salt thereof a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

## Description

The present invention relates to an orodispersible pharmaceutical solid dosage form of rasagiline or a pharmaceutically acceptable salt thereof having an improved dissolution and disintegration behavior, a process for the preparation of the orodispersible pharmaceutical solid dosage form, and its use in treating Parkinson's disease.

### Background Art

Parkinson's disease (PD) is one of the most common neurodegenerative disorders, which is estimated to affect 6.2 million people worldwide. In Europe, more than 1 million people live with Parkinson's disease, whereas in Japan, according to a study of the Japanese Society of Neurology, there are approximately 200,000 patients. However, these figures may be considerably higher as many people may go undiagnosed. As the incidence of Parkinson's disease rises significantly with age, and the life expectancy is getting higher, the prevalence of the disease is believed to rise dramatically in the near future - nearly 13 million people with Parkinson's by 2040. The economic impact of the disease is enormous - the annual European cost is estimated at 13.9 € billion.

Parkinson's disease is a progressive disorder, which can begin with mild stiffness and infrequent tremors, which progress over a period of ten or more years to frequent tremors and memory impairment, and ultimately, to uncontrollable tremors and dementia. The disease produces a slowly increasing disability in purposeful movement. Current drug treatments or therapies may include levodopa, carbidopa, bensarazide, entacapone, dopamine agonists like rotigotine, pramipexole, ropinirole, apomorphine, monoamine oxidase (MAO-B) inhibitors like selegiline, rasagiline, safinamide, tranylcypromine; COMT inhibitors like entacapone, tolcapone; and glutamate antagonists like amantadine. Unfortunately, such drug therapies frequently become less effective or ineffective over the time. In general, a PD patient may require multiple drugs in combination to extend the time period of efficacy of drug therapies.

Selective, irreversible MAO-B inhibitors are recommended due to their safety, tolerability, and easier clinical handling. Among available MAO-B inhibitors approved for therapeutic use, rasagiline is one of the most widespread used in the treatment of Parkinson's disease. In Europe, rasagiline (Azilect^{®}) was approved by the European Medicines Agency (EMA) in February 2005, indicated for the treatment of idiopathic Parkinson's disease as monotherapy (without levodopa) or as adjunct therapy (with levodopa) in patients with end-of-dose fluctuations. Azilect^{®} is only available as immediate release conventional film coated tablets in 1 mg strength, which according to the approved posology, shall be taken once daily either with or without levodopa. In Japan, Azilect^{®} was approved in March 2018, and is only available as immediate release conventional film coated tablets in 0.5 mg and 1 mg strength, for the same indication as in Europe.

The development of solid pharmaceutical dosage forms that disintegrate quickly in the mouth without requiring water is of high interest due to advantages that these dosage forms provide to patients suffering from difficulty in swallowing. Such condition is particularly important amongst elderly, stroke victims or patients affected by psychiatric disorders who refuse to swallow. A particular solid pharmaceutical dosage forms that rapidly disintegrates are orodispersible tablets (also named ODT: orally disintegrated tablet), which do not require water, and hence can be consumed in situations where patients require an easy-to-administer dosage form. Parkinsonian patients face swallowing difficulties and many patients tend to dribble, thus, hindering their treatment by reducing patient compliance. Accordingly, PD patients will be more likely to comply to dosage regimens if swallowing tablets or capsules is not required.

Aside from being rapidly dissolved in the oral cavity of a patient, fast disintegrating pharmaceutical dosage forms are known to be adequate for absorption of the active ingredient before reaching the stomach. Such absorption is accomplished by contact with the buccal, sublingual, pharyngeal and/or esophageal mucous membranes. Said early drug absorption facilitates not only pre-gastric absorption (predominantly buccal) but also a higher bioavailability because the active ingredient absorbed this way avoids pre-systemic metabolism, and thus, it permits, theoretically, the administration of lower doses.

However, when designing orodispersible pharmaceutical dosage forms, it should be borne in mind that it is a standard requirement by regulatory agencies to prove strict bioequivalence criteria of said orodispersible pharmaceutical dosage form versus the conventional film coated tablet of the reference product. As a consequence of the potential early drug absorption events of orodispersible pharmaceutical dosage forms, which generally occur in the buccal, sublingual, pharyngeal and/or esophageal mucous membranes, the requirement of bioequivalence poses considerable challenges for the development of fast disintegrating dosage forms.

For example, Comoglu et al. (Formulation, in vitro and in vivo evaluation of taste masked rasagiline orally fast disintegrating tablets (ODTS), Research & Reviews in Pharmacy and Pharmaceutical Sciences, 2017, vol. 6(2): 27-38) developed rasagiline ODT formulations, which were evaluated by means of an *in vivo* bioavailability study versus conventional rasagiline film coated immediate release tablets (Azilect^{®}). Rasagiline plasma exposure of the ODT formulations was revealed to be higher than the one obtained with conventional Azilect^{®} tablets (with the same dose). Such results obtained by the ODT formulations in the *in vivo* bioavailability study indicated that first-pass metabolism of rasagiline was decreased while, at the same time, it also indicated that rasagiline ODT formulations were suprabioequivalent with respect to conventional rasagiline film coated immediate release tablets (Azilect^{®}).

In another example, Tabi et al., (The pharmacokinetic evaluation of selegiline ODT for the treatment of Parkinson's disease, Expert Opin. Drug Metab. Toxicol., 2013; vol. 9(5): 629-36), studied the bioavailability of selegiline, which, as rasagiline, is also a MAO-B inhibitor, by comparing orally disintegrated tablets (ODT) containing 10 mg of selegiline with conventional tablets containing also 10 mg dose of selegiline as active ingredient. Selegiline plasma exposure of 10 mg orally disintegrated tablets (ODT) was found to be twelve-time higher than conventional selegiline 10 mg tablets. Additionally, it was found that about 30% of selegiline in the ODT form (10 mg) was absorbed through the mucous membrane of the mouth within 1 minute. Selegiline orally disintegrated tablets (ZELAPAR^{®}) are approved by the FDA only with 1.25 mg strengths, probably because of the higher suprabioavailability of orally disintegrated tablets containing 10 mg of selegiline.

In addition to the above, it is known that MAO enzyme is widely distributed in the body's tissues, with a high degree of expression in the gastro-intestinal tract. Therefore, it can be expected that an intestinal MAO binding would also affect rasagiline bioavailability in orally disintegrated pharmaceutical dosage forms as compared to conventional rasagiline film coated immediate release tablets (Azilect^{®}) - immediate release meaning rapid release of rasagiline at the stomach.

There are disclosures in the prior art which attempt to obtain fast disintegrating pharmaceutical compositions comprising rasagiline. For example, WO2012015946 discloses pharmaceutical forms of at least one polymeric pharmaceutical excipient and rasagiline or a pharmaceutically acceptable salt. Said document discloses that oral dosage forms of the invention relate to fast disintegrating formulations which provide a means to avoid the absorption of rasagiline in the stomach. To accomplish this, the fast disintegrating formulations were designed to rapidly disperse within the mouth to allow maximum contact of rasagiline with the buccal, sublingual, pharyngeal and/or esophageal mucous membranes, thus enhancing absorption of rasagiline by contact with such membranes.

WO2006057912 also discloses fast disintegrating formulations comprising rasagiline which are intended to minimize the absorption of rasagiline in the stomach by absorption of rasagiline into the buccal membrane before reaching the stomach. Accordingly, the use of particles having a non-filamentous microstructure of at least two sugar alcohols achieved such desired effect.

Another hurdle that shall be by-passed when developing orodispersible pharmaceutical dosage forms corresponds to the organoleptic properties of the active pharmaceutical ingredient. Many drugs are known to have unpleasant organoleptic properties, which may lead to lower patient compliance and adherence to the treatment. In this respect, it is known that pharmaceutical dosage forms comprising rasagiline may produce dry mouth as well as it may cause local numbness if they are absorbed from the buccal cavity. Therefore, the design of an orodispersible pharmaceutical solid dosage form of rasagiline should be able to overcome this challenge.

On the other hand, stability of pharmaceutical compositions of rasagiline and its pharmaceutically acceptable salts is known to be a matter of concern in the prior art. For example, document WO2013168032 reports that it is difficult to obtain stable formulations of rasagiline or its salts together with sugar alcohols. The document reveals that the main cause of the degradation are side reactions leading to oxidation of the active ingredient. In order to avoid formation of degradation products, WO2013168032 proposes the use of antioxidation excipients, such as ascorbic acid or cyclodextrins.

It is known in the art that the main degradation product of rasagiline is indan-1-amine, also known as rasagiline "impurity I". Indeed, said impurity also corresponds to the major metabolite of the active ingredient, which despite having neuroprotective capabilities, it does not inhibit monoamine oxidase B (Dimpfel, W. et al., Effects of rasagiline, its metabolite aminoindan and selegiline on glutamate receptor mediated signaling in the rat hippocampus slice in vitro, BMC Pharmacol., 2011, 11:2). Consequently, degradation of rasagiline into indan-1-amine impurity could even further reduce the efficacy of rasagiline pharmaceutical compositions in the treatment of Parkinson's disease.

Therefore, in view of the prior art, it exists the need to provide for orodispersible pharmaceutical dosage forms comprising rasagiline (or a pharmaceutically acceptable salt thereof) with all fast disintegrating-based advantages described above while at the same time having the bioavailability sufficiently equivalent to the conventional immediate release film coated tablets of the prior art (Azilect^{®}). Indeed, despite the attractiveness of fast disintegrating dosage forms for Parkinsonian patients, there has not been neither in Europe nor in Japan, up to date, any commercially available orodispersible dosage form comprising rasagiline, which highlights the difficult task of formulating rasagiline in orodispersible form being bioequivalent to the immediate release tablets of the reference product (Azilect^{®}). Furthermore, the requirement of providing orodispersible dosage forms of rasagiline which avoid the undesirable effect of dry mouth and local numbness when administered to a patient still remains as an unsolved challenge.

### Summary of the invention

The inventors have been capable of designing a new orodispersible pharmaceutical solid dosage form comprising a therapeutically effective amount of rasagiline or a pharmaceutically acceptable salt thereof and a pharmaceutically acceptable polymer suitable for forming a matrix with rasagiline or a pharmaceutically acceptable salt thereof, wherein rasagiline or a pharmaceutically acceptable salt thereof is trapped within the matrix formed with a pharmaceutically acceptable polymer, and wherein said orodispersible pharmaceutical solid dosage form exhibits a dissolution profile at buccal pH which minimizes or even substantially prevents dissolution of rasagiline or a pharmaceutically acceptable salt thereof in the buccal cavity, thereby avoiding issues such as dry mouth or local numbness.

Furthermore, the inventors have also surprisingly found that the composition of the present invention, wherein rasagiline or a pharmaceutically acceptable salt thereof is trapped within the matrix formed with a pharmaceutically acceptable polymer, provides precise control for allowing dissolution of the active ingredient mainly at stomach (gastric pH) whereas no substantial dissolution of the active is observed in the buccal cavity (buccal pH) and in the intestinal tract. In other words, the composition of the present invention, wherein rasagiline or a pharmaceutically acceptable salt thereof is trapped within the matrix formed with a pharmaceutically acceptable polymer, allows for adequate disintegration properties while ensuring minimal absorption of rasagiline through the buccal membrane and the intestinal tract but providing essentially total absorption of rasagiline in the stomach.

Overall, the specific dissolution profile and disintegrating behavior of the orodispersible pharmaceutical solid dosage form of the invention allows to provide for a bioavailability sufficiently equivalent to the one of the conventional immediate release tablets of the prior art (i.e. Azilect^{®}). In this respect, by being sufficiently equivalent, the orodispersible pharmaceutical solid dosage form of the invention avoids the risk of being suprabioequivalent, which would affect the toxicity and/or the efficacy of said orodispersible dosage form. Thus, orodispersible pharmaceutical solid dosage forms of the invention constitute a valuable therapeutic tool for Parkinsonian patients, which suffer from difficulty in swallowing (dysphagia) and could help those patients to improve their adherence to the treatment. The dosage form of the invention has the ability to disintegrate under the buccal cavity conditions of a human subject in less than three minutes , particularly in less than two minutes and a half, more particularly in less than two minutes , more particularly in less than one minute, and even more particularly in less than 45 seconds, before being swallowed. In particular, the dosage form of the invention disintegrates under the buccal cavity conditions of a human subject in the time range of from three minutes and five seconds, preferably in the time range of from two minutes and ten seconds, more preferably in the time range of from one minute and a half and fifteen seconds, and even more preferably in the time range of from one minute and twenty seconds. Thereby, the dosage forms of the invention shall be considered as orodispersible dosage forms, in accordance with the definition given by the European Pharmacopeia, edition 10.0, page 939.

Furthermore, the inventors, despite the teaching away comments from WO2013168032, have surprisingly found that the composition of the present invention, wherein rasagiline or a pharmaceutically acceptable salt thereof is trapped within the matrix formed with a pharmaceutically acceptable polymer, provides a new way of stabilizing rasagiline or a pharmaceutically acceptable salt, which prevents formation of degradation products over time; e.g. mainly indan-1-amine impurity (also known as "impurity I" of rasagiline), and therefore, the composition of the present invention comply with the criteria of impurities limit specifications required by the European or Japanese medicine regulatory agencies.

Thus, a first aspect of the present invention relates to an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

A second aspect of the invention relates to an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein the dosage form is obtainable by the following process: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form.

A third aspect of the present invention relates to an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix.

A fourth aspect of the invention relates to a process for the preparation of the dosage form as defined in the first, second and third aspects of the invention, which comprises: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form.

A fifth aspect of the invention relates to the dosage form as defined in the first aspect or the second aspect or the third aspect of the invention for use in the treatment of idiopathic Parkinson's disease as monotherapy (without levodopa) or as adjunct therapy (with levodopa) in patients with end of dose fluctuations.

### Brief description of the drawings

Figure 1 shows the dissolution profiles of a Film Coated Tablet (FCT) of Azilect^{®} versus Formulations 5, 6 and 7 as defined in Example 1 at buccal pH. Percentage of dissolution (%D) is expressed in % w/w; time (t) is expressed in minutes (min).
Figure 2 shows the dissolution profiles of a Film Coated Tablet (FCT) of Azilect^{®} versus Formulations 5, 6 and 7 as defined in Example 1 at gastric pH. Percentage of dissolution (%D) is expressed in % w/w; time (t) is expressed in minutes (min).

### Detailed description of the invention

The term "rasagiline" as used herein corresponds to the International Nonproprietary Name (INN) for R-(+)-N-propargyl-1-aminoindan but also as their pharmaceutically acceptable salts. Pharmaceutically acceptable salts of rasagiline comprise any of a broad range of inorganic and organic acids. Examples of such salts include acid addition salts with a mineral acid such as hydrochloric acid, hydrobromic acid, hydroiodic acid, sulfuric acid, nitric acid, or phosphoric acid, and those with an organic acid such as methanesulfonic acid, tartaric acid, oxalic acid, benzoic acid, galactaric acid, gluconic acid, glucuronic acid, p-toluene sulfonic acid, maleic acid, succinic acid, acetic acid, fumaric acid, benzenesulfonic acid, formic acid, propionic acid, malonic acid, lactic acid, malic acid, citric acid, carbonic acid, picric acid, ethanesulfonic acid, glutamic acid, tannic acid. The preferred salts for the purpose of the invention are rasagiline tartrate or rasagiline mesylate.

The term "polymer" as used herein is defined as any pharmaceutically acceptable polymer suitable for forming a matrix with rasagiline or a pharmaceutically acceptable salt thereof that modify the dissolution profile of rasagiline or pharmaceutically acceptable salts thereof. In particular, the polymer is able to form a matrix that allows rasagiline or a pharmaceutically acceptable salt thereof to be trapped within said formed matrix The term polymer as used herein comprises any of a broad range of matrix forming polymers including hydrophilic polymers, hydrophobic polymers and water insoluble polyelectrolytes such as ion exchange resins including sulfonated polystyrene polymers, sulfonated styrene-divinylbenzene copolymers, polymethacrylic acid polymers, methacrylic acid-divinylbenzene copolymers, amongst others.

The term "ion exchange resin" as used herein is defined as any pharmaceutically acceptable water insoluble polymer that can exchange their mobile ions with the ions in the surrounding medium. The term as used herein comprises sodium or potassium or magnesium or calcium or iron or zinc salts or partial sodium or potassium or magnesium or calcium or iron or zinc salts of sulfonated polystyrene polymers, sulfonated styrene-divinylbenzene copolymers, polymethacrylic acid polymers, methacrylic acid-divinylbenzene copolymers, amongst others. The term as used herein also comprises protonated sulfonated polystyrene polymers, protonated sulfonated styrene-divinylbenzene copolymers, protonated polymethacrylic acid polymers, protonated methacrylic acid-divinylbenzene copolymers, amongst others.

The term "cation exchange resin" as used herein is defined as any pharmaceutically acceptable water insoluble polymer that can exchange their mobile cations with the cations in the surrounding medium. The term as used herein comprises sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, amongst others. The term as used herein also comprises protonated sulfonated polystyrene polymers, protonated sulfonated styrene-divinylbenzene copolymers, protonated polymethacrylic acid polymers, protonated methacrylic acid-divinylbenzene copolymers, amongst others.

The term "acidic cation exchange resin" as used herein, is defined as any pharmaceutically acceptable water insoluble polymer that can exchange their mobile cations with the cations in the surrounding medium and have acidic functional groups such as sulfonic or carboxylic groups. The term as used herein comprises sodium or potassium or magnesium or calcium salts or partial sodium or potassium or magnesium or calcium salts of sulfonated polystyrene polymers, sulfonated styrene-divinylbenzene copolymers, amongst others. The term as used herein also comprises protonated sulfonated polystyrene polymers, protonated sulfonated styrene-divinylbenzene copolymers, protonated polymethacrylic acid polymers, protonated methacrylic acid-divinylbenzene copolymers, amongst others.

The term "weakly acidic cation exchange resin" as used herein, is defined as any pharmaceutically acceptable water insoluble polymer that can exchange their mobile cations with the cations in the surrounding medium and have weakly acidic functional groups such as carboxylic groups. The term as used herein comprise sodium or potassium or magnesium or calcium salts or partial sodium or potassium or magnesium or calcium salts of polymethacrylic acid polymers, methacrylic acid-divinylbenzene copolymers, amongst others.

The term "polacrilin potassium" as used herein is defined as a partial potassium salt of a methacrylic acid-divinylbenzene copolymer wherein a weakly acidic salt is formed from the carboxylic acid functional groups of the methacryclic acid. Hereinafter, the term "partial polacrilin potassium" is commercialized under several commercial names such as Purolite^{®} C115KMR, Amberlite^{®} IRP88, amongst others.

The term "polacrilin sodium" as used herein is defined as a partial sodium salt of a sulfonated styrene-divinylbenzene copolymer wherein an acidic salt is formed from the sulfonic acid functional groups of the styrene. Hereinafter, the term "partial polacrilin sodium" is commercialized under several commercial names such as Purolite^{®} C100NaMR, amongst others.

The term "resin IR-120" as used herein is defined as a protonated form of a sulfonated styrene-divinylbenzene copolymer. Hereinafter, the term "resin IR-120" is commercialized under several commercial names such as Amberlite^{®} IR-120, amongst others.

The term "matrix" as used herein is defined as the combination of a polymer and rasagiline or pharmaceutically acceptable salts thereof, wherein the rasagiline or the pharmaceutically acceptable salts thereof are bound to the polymer by intermolecular forces such as electrostatic interactions, hydrogen bonding and van der Waal forces, amongst others. Apparently due to these forces the matrix allows rasagiline or a pharmaceutically acceptable salt thereof to be trapped within said formed matrix. Particularly, wherein the polymer is an ion exchange resin, the rasagiline or pharmaceutically acceptable salts thereof are primarily bound by electrostatic interactions. More particularly, wherein the polymer is a cation exchange resin, rasagiline or pharmaceutically acceptable salts thereof is primarily bound by electrostatic interactions to the negatively charged functional groups of the cation exchange resin. More particularly, wherein the polymer is an acidic cation exchange resin, rasagiline or pharmaceutically acceptable salts thereof is primarily bound by electrostatic interactions to the negatively charged acidic functional groups of the acidic cation exchange resin. Even more particularly, wherein the polymer is polymethacrylic acid polymer, rasagiline or pharmaceutically acceptable salts thereof is primarily bound by electrostatic interactions to the negatively charged carboxylic acid groups of the polymethacrylic acid polymer. Even more particularly, wherein the polymer is sulfonated polystyrene polymer, rasagiline or pharmaceutically acceptable salts thereof is primarily bound by electrostatic interactions to the negatively charged sulfonic acid groups of the sulfonated polystyrene polymer. Even more particularly, wherein the polymer is sulfonated styrene-divinylbenzene copolymer, rasagiline or pharmaceutically acceptable salts thereof is primarily bound by electrostatic interactions to the negatively charged sulfonic acid groups of the sulfonated styrene-divinylbenzene copolymer.

The term "trapped within the matrix" means that basically all of the rasagiline or the pharmaceutically acceptable salt thereof is contained within the matrix. In other words, basically no rasagiline or the pharmaceutically acceptable salt thereof is present outside the matrix. In this way the improved dissolution and disintegration characteristics of the orodispersible solid dosage form can be obtained. "Basically all" means preferably more than 95%, more preferably more than 97%, and even more preferably more than 98%. It is preferred that basically all of the rasagiline or the pharmaceutically acceptable salt thereof is contained within the matrix homogeneously. In other words, rasagiline or the pharmaceutically acceptable salt thereof contained within the matrix is distributed throughout the matrix uniformly.

The term "orodispersible tablet" as used herein is defined in accordance with the European Pharmacopeia, edition 10.0, page 939 as an uncoated tablet intended to be placed in the mouth where it disperses rapidly before being swallowed, more precisely orodispersible tablets disintegrate within three minutes in the disintegration test. In accordance with this definition the term orodispersible tablet is intended to be a synonym of solid oral dosage forms named as orodispersible tablet, orally disintegrating or disintegrated tablet (ODT), fast disintegrating tablet, fast dissolving tablet and chewable tablet, amongst others.

The term "buccal pH" as used herein is defined as the pH found in saliva of a healthy human subject. Such pH normally varies from about 6.5 to about 7.5. The term "buccal pH" as used herein is defined as pH about 7.0.

The term "gastric pH" as used herein is defined as the pH found in the stomach of a healthy human subject in the fasted-state. Such pH typically varies from about 1 to about 3, preferably from about 1 to about 2.5, more preferably from about 1 to about 2, and even more preferably from about 1 to about 1.6. The term "gastric pH" as used herein is defined as pH about 1.2.

The term "disintegrate" as used herein is defined as the action whereby a solid dosage form is brought from a solid state to a state of complete disintegration.

The term "complete disintegration" as used herein is defined in accordance with the European Pharmacopeia, edition 10.0, page 323, where it is defined as that state in which any residue of the dosage form, except insoluble fragments, remaining on the screen of the test apparatus or adhering to the lower surface of the discs, if used, is a soft mass having no palpably firm core. Disintegration, as defined herein, does not imply complete dissolution of the dosage form or even of its active pharmaceutical ingredient. Ideally complete disintegration in the buccal cavity is accompanied by less than 15%, preferably less than 12% and more preferably less than 10%, and up to (almost) no dissolution of rasagiline or its pharmaceutically acceptable salts in the buccal cavity.

The term "dissolution profile" as used herein refers to dissolution over time of rasagiline from the dosage form of the invention. Hereinafter, the dissolution profile is measured in weight of dissolved rasagiline per initial weight of rasagiline in the dosage form, and it is expressed in weight percentage (% w/w). Unless otherwise stated, hereinafter, the dissolution profiles at buccal pH are determined using a European Pharmacopeia Apparatus II (paddles), placing the dosage form in 500 mL, under pH=7.0, at 37 °C and stirring at 50 revolutions per minute. Unless otherwise stated, hereinafter, the dissolution profiles at gastric pH are determined using an orbital bath, placing the dosage form in 50 mL, under pH=1.2, at 37 °C and stirring at 100 revolutions per minute.

The term "normal storage conditions" as used herein is defined as conditions of temperature from 20 to 30°C and relative humidity from 55 to 65%.

The term "intragranular" as used herein is referred to the components of the formulation added in the process steps before granulating.

The term "extragranular" as used herein is referred to the components added in the process steps after granulating.

The terms "degradation products" or "degradation impurities" as used herein are defined as those impurities resulting from a chemical change in the drug substance brought about during manufacture and/or storage of the new drug product by the effect of, for example, light, temperature, atmospheric oxygen, water vapor, or by reaction with an excipient and/or the container closure system.

Hereinafter, the amount of indan-1-amine, also known as rasagiline "impurity I", present in the dosage form of the invention is expressed in weight of indan-1-amine per initial weight of rasagiline in the dosage form, and this amount is given in weight percentage (% w/w).

The term "pharmaceutically acceptable excipient" (also named as "excipients") refers to a substance formulated alongside with the active pharmaceutical ingredient of a medicinal product and includes all kind of pharmaceutically acceptable compounds commonly used in pharmaceutical compositions and in particular orodispersible tablets. The term "pharmaceutically acceptable excipients" comprise diluents, lubricants, disintegrants, surfactants, flavouring agents, sweeteners, glidants, antiadherants and mixtures thereof.

The term "diluent" as used herein is defined as a pharmaceutical acceptable excipient that is used as diluent in pharmaceutical compositions. The term "diluent" comprises one or combinations of two or more selected from the group of mannitol, maltol, sorbitol, maltitol, xylitol, isomalt, erythritol, lactose, preferably lactose monohydrate, starch and its derivatives, cellulose and its derivatives, in particular microcrystalline cellulose.

The term "lubricant" as used herein is defined as a pharmaceutical acceptable excipient that is used as lubricant in pharmaceutical compositions. The term "lubricant" comprises one or combinations of two or more selected from the group of talc, sodium benzoate, sodium stearyl fumarate, calcium stearate, magnesium stearate, zinc stearate, glyceryl behenate, stearic acid and glyceryl monostearate; more particularly, such lubricant is selected from the group of sodium stearyl fumarate and magnesium stearate.

The term "disintegrant" as used herein is defined as a pharmaceutical acceptable excipient that is used as disintegrant in pharmaceutical compositions. The term "disintegrant" comprises one or combinations of two or more selected from the group of croscarmellose sodium, crospovidone, carmellose sodium, carmellose calcium, corn starch, sodium starch glycolate and pregelatinized starch; more particularly, such disintegrants are selected from the group of croscarmellose sodium, crospovidone and sodium starch glycolate.

The term "flavouring agent" as used herein is defined as a pharmaceutical acceptable excipient that is used as flavouring agent in pharmaceutical compositions. The term "flavouring agent" comprises one or combinations of two or more selected from the group of cherry, raspberry, apricot, pear, strawberry, bitter masker, pineapple, lemon, honey, mint garden, orange, peppermint, menthol, black currant, banana, red fruits, wild berries and caramel flavour. The preferred flavouring agents for the purpose of the invention are cherry, raspberry, apricot, pear, strawberry, bitter masker, pineapple, lemon and honey flavour.

The term "sweetener" as used herein is defined as a pharmaceutical acceptable excipient that is used as sweetener in pharmaceutical compositions. The term "sweetener" comprises one or combinations of two or more selected from the group of aspartame, potassium acesulfame (Acesulfame K), sodium saccharinate, neohesperidine dihydrochalcone, sucralose, sucrose, fructose and monoammonium glycyrrhizinate.

The term "friability" as used herein refers to the tendency for a tablet to chip, crumble or break during handling. The test of friability is carried out following the guidelines of the European Pharmacopeia, edition 10.0, pp. 336-337. A maximum loss of mass not greater than 1.0 % is considered acceptable for most products.

As it is mentioned above, an aspect of the invention relates to an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 8% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 5% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 85% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 85% w/w of the rasagiline relative to the total rasagiline content of the dosage form is dissolved, and (iii) after 40 minutes at pH=1.2, at least 95% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 75% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 75% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 95% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 95% w/w of the rasagiline relative to the total rasagiline content of the dosage form is dissolved, and
wherein said orodispersible pharmaceutical solid dosage form is disintegrated in less than 3 minutes.

In an embodiment, the polymer of the invention is an ion exchange resin, preferably a cation exchange resin, more preferably an acidic or weakly acidic cation exchange resin. In another embodiment the polymer of the invention is selected from sulfonated polystyrene polymers, sulfonated styrene-divinylbenzene copolymers, polymethacrylic acid polymers and methacrylic acid-divinylbenzene copolymers. In an embodiment, the polymer of the invention is selected from a partial sodium or potassium or magnesium or calcium or iron or zinc salt of sulfonated polystyrene polymer, sulfonated styrene-divinylbenzene copolymer, polymethacrylic acid polymer and methacrylic acid-divinylbenzene copolymer. In another embodiment, the polymer of the invention is selected from a protonated sulfonated polystyrene polymer, protonated sulfonated styrene-divinylbenzene copolymer, protonated polymethacrylic acid polymer and protonated methacrylic acid-divinylbenzene copolymer. In another embodiment, the polymer of the invention is selected from a partial sodium or potassium or magnesium or calcium salts of sulfonated polystyrene polymer, sulfonated styrene-divinylbenzene copolymer, polymethacrylic acid polymers and methacrylic acid-divinylbenzene copolymer. In a particular embodiment, the polymer of the invention is a partial potassium salt of methacrylic acid-divinylbenzene copolymer. In a more particular embodiment, the polymer of the invention is polacrilin potassium. In a particular embodiment, the polymer of the invention is a partial sodium salt of sulfonated styrene-divinylbenzene copolymer. In a more particular embodiment, the polymer of the invention is polacrilin sodium.

In a particular embodiment, the polymer of the invention is as a protonated form of a sulfonated styrene-divinylbenzene copolymer.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 8% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 5% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 85% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 85% w/w of the rasagiline relative to the total rasagiline content of the dosage form is dissolved, and (iii) after 40 minutes at pH=1.2, at least 95% w/w of the rasagiline relative to the total rasagiline content of the dosage form is dissolved, and wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 75% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 75% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 95% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 90% w/w of the rasagiline relative to the total rasagiline content of the dosage form is dissolved, and
wherein said orodispersible pharmaceutical solid dosage form is disintegrated in less than 3 minutes.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 12% w/w of the rasagiline relative to the total rasagiline content of the dosage form is dissolved, and
   (ii) after 4 minutes, less than 18% w/w of the rasagiline relative to the total rasagiline content of the dosage form is dissolved, and
   (iii) after 6 minutes, less than 20% w/w of the rasagiline relative to the total rasagiline content of the dosage form is dissolved, and
(b) at pH=1.2
   (i) after 15 minutes, more than 85% w/w of the rasagiline relative to the total rasagiline content of the dosage form is dissolved, and
   (ii) after 40 minutes, more than 95% w/w of the rasagiline relative to the total rasagiline content of the dosage form is dissolved, and
wherein said orodispersible pharmaceutical solid dosage form is disintegrated in less than 3 minutes.

In an embodiment, the solid oral dosage form of the invention disintegrates at buccal pH in less than three minutes, preferably in less than two minutes and a half, preferably in less than two minutes, more preferably in less than one minute, and even more preferably in less than 45 seconds.

In an embodiment, the solid oral dosage form of the invention disintegrates at buccal pH in the time range of from three minutes and five seconds, preferably in the time range of from two minutes and ten seconds, more preferably in the time range of from one minute and a half and fifteen seconds, and even more preferably in the time range of from one minute and twenty seconds.

In an embodiment, the orodispersible pharmaceutical solid dosage form of the invention is an orodispersible tablet. In another embodiment, the orodispersible pharmaceutical solid dosage form of the invention is an orally disintegrating or disintegrated tablet (ODT). In another embodiment, the orodispersible pharmaceutical solid dosage form of the invention is a fast disintegrating tablet. In another embodiment, the orodispersible pharmaceutical solid dosage form of the invention is a fast dissolving tablet. In another embodiment, the orodispersible pharmaceutical solid dosage form of the invention is a chewable tablet. In another embodiment, the orodispersible pharmaceutical solid dosage form of the invention is an orodispersible chewable tablet. In another embodiment, the orodispersible pharmaceutical solid dosage form of the invention is in the form of orodispersible granules. In another embodiment, the orodispersible pharmaceutical solid dosage form of the invention is in the form of orodispersible powder particles. In another embodiment, the orodispersible pharmaceutical solid dosage form of the invention is in the form of an oral thin film.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersible pharmaceutical solid dosage form of the invention is a tablet which comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, an acidic cation exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, a weakly acidic cation exchange resin suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, wherein the ion exchange resin suitable for forming a matrix with rasagiline or a pharmaceutically acceptable salt thereof is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, a partial potassium salt of a methacrylic acid-divinylbenzene copolymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, a partial sodium salt of a sulfonated styrene-divinylbenzene copolymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline or a pharmaceutically acceptable salt thereof, polacrilin potassium suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In a preferred embodiment, the orodispersable tablet of the invention comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In a preferred embodiment, the orodispersable tablet of the invention comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which (i) after 2 minutes at pH=7.0, less than 12% w/w of the rasagiline relative to the total rasagiline content of the dosage form is dissolved, and (ii) after 15 minutes at pH=1.2, more than 75% w/w of the rasagiline relative to the total rasagiline content of the dosage form is dissolved, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds.

In a preferred embodiment, the orodispersable tablet of the invention comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, and wherein said dosage form exhibits a dissolution profile according to which
(a) at pH=7.0
   (i) after 2 minutes, less than 15% w/w of the rasagiline relative to the total rasagiline content of the dosage form is dissolved, and
   (ii) after 4 minutes, less than 20% w/w of the rasagiline relative to the total rasagiline content of the dosage form is dissolved, and
   (iii) after 6 minutes, less than 25% w/w of the rasagiline relative to the total rasagiline content of the dosage form is dissolved, and
(b) at pH=1.2
   (i) after 15 minutes, more than 75% w/w of the rasagiline relative to the total rasagiline content of the dosage form is dissolved, and
   (ii) after 40 minutes, more than 90% w/w of the rasagiline relative to the total rasagiline content of the dosage form is dissolved, and
and wherein said orodispersible pharmaceutical solid dosage form is disintegrated in less than two minutes and a half, preferably in less than two minutes, more preferably in less than one minute, and even more preferably in less than 45 seconds.

The inventors have also found that orodispersible pharmaceutical solid dosage forms of the invention comprising a pharmaceutically acceptable polymer suitable for forming a matrix with rasagiline or a pharmaceutically acceptable salt thereof diminish the formation of degradation products, such as indan-1-amine impurity (also known as "impurity I" of rasagiline). Hence, the present invention prevents the formation of degradation products over time.

In an embodiment, an orodispersible tablet of the invention, after one month of storage at a temperature 25 °C and at a relative humidity 60%, contains indan-1-amine impurity below quantification limit.

In an embodiment, an orodispersible tablet of the invention, after one month of storage at a temperature 40 °C and at a relative humidity 75%, contains indan-1-amine impurity below 0.05%. In another embodiment, an orodispersible tablet of the invention, after three months of storage at a temperature 40 °C and at a relative humidity 75%, contains indan-1-amine impurity less than 0.30%, preferably less than 0.25% and more preferably less than 0.20%.

The amount of rasagiline per orodispersible pharmaceutical dosage form is of from 0.05 mg to 5.0 mg, calculated based on the weight of rasagiline free base. In an embodiment, the amount of rasagiline per orodispersible pharmaceutical dosage form is of from 0.1 mg to 4.5 mg, preferably of from 0.25 mg to 3.0 mg, more preferably of from 0.5 mg to 2.0 mg, and even more preferably of from 0.5 mg to 1.5 mg. In a preferred embodiment, the amount of rasagiline per orodispersible pharmaceutical dosage form is of from 0.5 mg to 1.0 mg. In a more preferred embodiment, the amount of rasagiline per orodispersible tablet is of from 0.5 mg to 1.0 mg, calculated based on the weight of rasagiline free base. Hereinafter, all the percentages related to the amount of a certain component in the dosage form are given as the ratio between the weight of the component and the total weight of the dosage form and are expressed by the term "% by weight of the component in the dosage form".

In another embodiment, the orodispersible pharmaceutical dosage form of the invention comprises rasagiline in an amount of from 0.10 to 2.00%, calculated based on the weight of rasagiline free base, preferably of from 0.20 to 1.75%, more preferably of from 0.25 to 1.50%, and even more preferably of from 0.25 to 1.25%. In a preferred embodiment, orodispersible pharmaceutical dosage form of the invention comprises rasagiline in an amount of from 0.90 to 1.10%. In a more preferred embodiment, the orodispersible tablet of the invention comprises rasagiline in an amount of from 0.10 to 2.00%, calculated based on the weight of rasagiline free base.

In an embodiment, the orodispersable tablet of the invention comprises a therapeutically effective amount of rasagiline tartrate, an acidic orweakly acidic cation exchange resin suitable for forming a matrix with rasagiline tartrate, and one or more pharmaceutically acceptable excipients, wherein rasagiline tartrate is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In a preferred embodiment, the orodispersable tablet of the invention comprises a therapeutically effective amount of rasagiline tartrate, polacrilin sodium suitable for forming a matrix with rasagiline tartrate, and one or more pharmaceutically acceptable excipients, wherein rasagiline tartrate is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline tartrate, wherein rasagiline tartrate has a particle size distribution (PSD) such that D(v, 10) is more than 3 µm and less than or equal to 40 µm, preferably is more than 8 µm and less than or equal to 30 µm, more preferably is more than 10 µm and less than or equal to 20 µm, even more preferably is more than 13 µm and less than or equal to 18 µm; and/or D(v, 50) is more than 45 µm and less than or equal to 120 µm, preferably is more than 60 µm and less than or equal to 105 µm, more preferably is more than 70 µm and less than or equal to 95 µm, even more preferably is more than 77 µm and less than or equal to 85 µm; and/or D(v, 90) is more than 275 µm and less than or equal to 400 µm, preferably is more than 290 µm and less than or equal to 380 µm, more preferably is more than 305 µm and less than or equal to 360 µm, even more preferably is more than 320 µm and less than or equal to 350 µm.

In an embodiment, the orodispersable tablet of the invention comprises a therapeutically effective amount of rasagiline mesylate, an acidic or weakly acidic cation exchange resin suitable for forming a matrix with rasagiline mesylate, and one or more pharmaceutically acceptable excipients, wherein rasagiline mesylate is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In a preferred embodiment, the orodispersable tablet of the invention comprises a therapeutically effective amount of rasagiline mesylate, polacrilin sodium suitable for forming a matrix with rasagiline mesylate, and one or more pharmaceutically acceptable excipients, wherein rasagiline mesylate is trapped within the matrix, and wherein said dosage form exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than two minutes and a half, preferably in less than two minutes, more preferably in less than one minute, and even more preferably in less than 45 seconds, wherein disintegration test was performed using a European Pharmacopeia disintegration apparatus A, placing the dosage form in water having pH=7 at 37 °C and 30 cycles per minute.

In an embodiment, the orodispersable tablet of the invention comprises a therapeutically effective amount of rasagiline mesylate, wherein rasagiline mesylate has a particle size distribution (PSD) such that D(v, 10) is more than 3 µm and less than or equal to 40 µm; and/or D(v, 50) is more than 45 µm and less than or equal to 120 µm; and/or D(v, 90) is more than 275 µm and less than or equal to 400 µm.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, wherein rasagiline or a pharmaceutically acceptable salt thereof is trapped within the matrix, and wherein not more than 3% w/w of rasagiline or pharmaceutically acceptable salt thereof present in the orodispersible pharmaceutical solid dosage form is not trapped within the matrix formed with such pharmaceutically acceptable polymer.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, wherein rasagiline or a pharmaceutically acceptable salt thereof is trapped within the matrix, and wherein at least 97% w/w of rasagiline or pharmaceutically acceptable salt thereof present in the orodispersible pharmaceutical solid dosage form is trapped within the matrix formed with such pharmaceutically acceptable polymer.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, wherein rasagiline or a pharmaceutically acceptable salt thereof is trapped within the matrix, and wherein not more than 3% w/w of rasagiline or pharmaceutically acceptable salt thereof present in the orodispersible pharmaceutical solid dosage form is not trapped within the matrix formed with such ion exchange resin.

In an embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, wherein rasagiline or a pharmaceutically acceptable salt thereof is trapped within the matrix, and wherein at least 97% w/w of rasagiline or pharmaceutically acceptable salt thereof present in the orodispersible pharmaceutical solid dosage form is trapped within the matrix formed with such ion exchange resin.

In a particular embodiment, the orodispersible tablet of the invention comprises a therapeutically effective amount of rasagiline or a pharmaceutically acceptable salt thereof, an acidic or weakly acidic cation exchange resin suitable for forming a matrix with rasagiline or a pharmaceutically acceptable salt thereof, wherein rasagiline or a pharmaceutically acceptable salt thereof is trapped within the matrix, and wherein not more than 3% w/w of rasagiline or pharmaceutically acceptable salt thereof present in the orodispersible pharmaceutical solid dosage form is not trapped within the matrix formed with such acidic or weakly acidic cation exchange resin, and wherein said orodispersible tablet exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In a particular embodiment, the orodispersible tablet of the invention comprises a therapeutically effective amount of rasagiline or a pharmaceutically acceptable salt thereof, an acidic or weakly acidic cation exchange resin suitable for forming a matrix with rasagiline or a pharmaceutically acceptable salt thereof, wherein rasagiline or a pharmaceutically acceptable salt thereof is trapped within the matrix, and wherein at least 97% w/w of rasagiline or pharmaceutically acceptable salt thereof present in the orodispersible pharmaceutical solid dosage form is trapped within the matrix formed with such acidic or weakly acidic cation exchange resin, and wherein said orodispersible tablet exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In a more particular embodiment, the orodispersible tablet of the invention comprises a therapeutically effective amount of rasagiline or a pharmaceutically acceptable salt thereof, polacrilin sodium suitable for forming a matrix with rasagiline or a pharmaceutically acceptable salt thereof, wherein rasagiline or a pharmaceutically acceptable salt thereof is trapped within the matrix, and wherein not more than 3% w/w of rasagiline or pharmaceutically acceptable salt thereof present in the orodispersible pharmaceutical solid dosage form is not trapped within the matrix formed with polacrilin sodium, and wherein said orodispersible tablet exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In a more particular embodiment, the orodispersible tablet of the invention comprises a therapeutically effective amount of rasagiline or a pharmaceutically acceptable salt thereof, polacrilin sodium suitable for forming a matrix with rasagiline or a pharmaceutically acceptable salt thereof, wherein rasagiline or a pharmaceutically acceptable salt thereof is trapped within the matrix, and wherein at least 97% w/w of rasagiline or pharmaceutically acceptable salt thereof present in the orodispersible pharmaceutical solid dosage form is trapped within the matrix formed with polacrilin sodium, and wherein said orodispersible tablet exhibits a dissolution profile according to which (i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

The amount of the pharmaceutically acceptable polymer suitable for forming a matrix with rasagiline or a pharmaceutically acceptable salt thereof of the present invention is not particularly limited as long as the rasagiline and the pharmaceutically acceptable polymer form a matrix that has the dissolution profiles detailed above.

The weight-to-weight ratio of rasagiline or a pharmaceutically acceptable salt thereof to pharmaceutically acceptable polymer suitable for forming a matrix is from 0.5:1 to 1:10, preferably from 1:1 to 1:8, more preferably from 1:1 to 1:6, and even more preferably from 1:2 to 1:6.

In another embodiment, the weight-to-weight ratio of rasagiline or a pharmaceutically acceptable salt thereof to an ion exchange resin suitable for forming a matrix is from 0.5:1 to 1:10, preferably from 1:1 to 1:8, more preferably from 1:1 to 1:6, and even more preferably from 1:2 to 1:6.

In another embodiment, the weight-to-weight ratio of rasagiline or a pharmaceutically acceptable salt thereof to an acidic or weakly acidic cation exchange resin suitable for forming a matrix is from 0.5:1 to 1:10, preferably from 1:1 to 1:8, more preferably from 1:1 to 1:6, and even more preferably from 1:2 to 1:6.

In another embodiment, the weight-to-weight ratio of rasagiline or a pharmaceutically acceptable salt thereof to partial potassium salt of a polymethacrylic acid polymer suitable for forming a matrix is from 0.5:1 to 1:10, preferably from 1:1 to 1:8, more preferably from 1:1 to 1:6, and even more preferably from 1:2 to 1:6.

In another embodiment, the weight-to-weight ratio of rasagiline or a pharmaceutically acceptable salt thereof to partial sodium salt of a sulfonated polystyrene polymer suitable for forming a matrix is from 0.5:1 to 1:10, preferably from 1:1 to 1:8, more preferably from 1:1 to 1:6, and even more preferably from 1:2 to 1:6.

In a particular embodiment, the orodispersible pharmaceutical solid dosage form of the invention is an orodispersible tablet which comprises partial sodium salt of a sulfonated polystyrene polymer suitable for forming a matrix is from 0.5:1 to 1:10, preferably from 1:1 to 1:8, more preferably from 1:1 to 1:6, and even more preferably from 1:2 to 1:6.

In another embodiment, the orodispersible pharmaceutical solid dosage form of the invention comprises a pharmaceutically acceptable polymer suitable for forming a matrix in an amount from 0.5% to 10% by weight of the pharmaceutically acceptable polymer in the dosage form, preferably from 1.0% to 8.0%, more preferably from 1.5% to 7%, even more preferably from 2.0% to 6.0%.

In another embodiment, the orodispersible pharmaceutical solid dosage form of the invention comprises a an ion exchange resin suitable for forming a matrix in an amount from 0.5% to 10% by weight of the an ion exchange resin in the dosage form, preferably from 1.0% to 8.0%, more preferably from 1.5% to 7%, even more preferably from 2.0% to 6.0%.

In another embodiment, the orodispersible pharmaceutical solid dosage form of the invention comprises an acidic or weakly acidic cation exchange resin suitable for forming a matrix in an amount from 0.5% to 10% by weight of the acidic or weakly acidic cation exchange resin, preferably from 1.0% to 8.0%, more preferably from 1.5% to 7%, even more preferably from 2.0% to 6.0%.

In another embodiment, the orodispersible pharmaceutical solid dosage form of the invention comprises partial potassium salt of a polymethacrylic acid polymer suitable for forming a matrix in an amount from 0.5% to 10% by weight of the partial potassium salt of a polymethacrylic acid polymer, preferably from 1.0% to 8.0%, more preferably from 1.5% to 7%, even more preferably from 2.0% to 6.0%.

In another embodiment, the orodispersible pharmaceutical solid dosage form of the invention comprises partial sodium salt of a sulfonated polystyrene polymer suitable for forming a matrix in an amount from 0.5% to 10% by weight of the partial sodium salt of a sulfonated polystyrene polymer, preferably from 1.0% to 8.0%, more preferably from 1.5% to 7%, even more preferably from 2.0% to 6.0%.

In a particular embodiment, the orodispersible pharmaceutical solid dosage form of the invention is an orodispersible tablet which comprises partial sodium salt of a sulfonated polystyrene polymer suitable for forming a matrix in an amount from 0.5% to 10% by weight of the partial sodium salt of a sulfonated polystyrene polymer, preferably from 1.0% to 8.0%, more preferably from 1.5% to 7%, even more preferably from 2.0% to 6.0%.

The solid oral dosage form of the present invention may further comprise suitable pharmaceutically acceptable excipient, such as diluents, disintegrants, glidants, lubricants, as well as colouring agents, flavours and sweetening agents.

The solid oral dosage form of the present invention may comprise one or more suitable diluents. Suitable diluents for the oral dosage form are for example, sugars like mannitol, maltol, sorbitol, maltitol, xylitol, isomalt, erythritol, lactose, preferably lactose monohydrate, starch and its derivatives, and cellulose and its derivatives, in particular microcrystalline cellulose, amongst others.

In an embodiment, the orodispersible tablet of the invention comprises from 1 to 90% by weight of a diluent in the tablet, preferably from 5 to 90%, more preferably from 20 to 80%, even ore preferably from 50 to 80%.

In a particular embodiment, the orodispersible tablet of the invention comprises from 1 to 90% by weight of mannitol or lactose or mixtures thereof in the tablet, preferably from 5 to 90%, more preferably from 20 to 80%, even ore preferably from 50 to 80%.

The solid oral dosage form of the present invention may further comprise one or more lubricants. Suitable lubricants are for example stearic acid and derivatives thereof, such as calcium stearate, sodium stearyl fumarate or magnesium stearate, amongst others.

In an embodiment, the orodispersible tablet of the invention comprises from 0.01 to 2% by weight of lubricant in the tablet, preferably from 0.1 to 1.8%, more preferably from 0.2 to 1.6%.

In a particular embodiment, the orodispersible tablet of the invention comprises from 0.01 to 2% by weight of sodium stearyl fumarate in the tablet, preferably from 0.1 to 1.8%, more preferably from 0.2 to 1.6%.

The solid oral dosage form of the present invention may further comprise one or more disintegrants. The amount and the identity of the disintegrant are not particularly limited as long as the solid oral dosage form of the invention is disintegrated at buccal pH in less than three minutes, preferably in less than two minutes and a half, preferably in less than two minutes, more preferably in less than one minute, and even more preferably in less than 45 seconds, before being swallowed.

In an embodiment, the disintegrant is selected from the group of croscarmellose sodium, crospovidone, carmellose sodium, carmellose calcium, corn starch, sodium starch glycolate and pregelatinized starch and mixtures thereof. In a particular embodiment, the disintegrant is selected from the group of croscarmellose sodium, crospovidone and sodium starch glycolate and mixtures thereof. In a more particular embodiment, the disintegrant is selected from the group of croscarmellose sodium, crospovidone and sodium starch glycolate.

In an embodiment, the orodispersible tablet of the invention comprises one or more disintegrants in an amount from 1 to 35 % by weight of disintegrant in the orodispersible tablet, preferably from 5 to 25%, more preferably from 10 to 20%.

In a particular embodiment, the orodispersible tablet of the invention comprises crospovidone in an amount from 1 to 35 % by weight of crospovidone in the orodispersible tablet, preferably from 5 to 25%, more preferably from 10 to 20%.

In a particular embodiment, the orodispersible tablet of the invention comprises croscarmellose sodium in an amount from 1 to 35 % by weight of croscarmellose sodium in the orodispersible tablet, preferably from 5 to 25%, more preferably from 10 to 20%.

In a particular embodiment, the orodispersible tablet of the invention comprises sodium starch glycolate in an amount from 1 to 35 % by weight of sodium starch glycolate in the orodispersible tablet, preferably from 5 to 25%, more preferably from 10 to 20%.

The solid oral dosage form of the present invention may comprise intragranular and extragranular disintegrant excipients. The amount and the identity of the intragranular and extragranular disintegrant excipients are not particularly limited as long as the solid oral dosage form of the invention is disintegrated at buccal pH in less than three minutes, preferably in less than two minutes and a half, preferably in less than two minutes, more preferably in less than one minute, and even more preferably in less than 45 seconds, before being swallowed.

In a particular embodiment, the orodispersible tablet of the invention comprises one or more disintegrants wherein said one or more disintegrants are intragranular, i.e. added in the process steps before granulating. In a particular embodiment, the orodispersible tablet of the invention comprises crospovidone intragranular, in an amount from 1 to 35 % by weight of crospovidone in the orodispersible tablet, preferably from 5 to 25%, more preferably from 10 to 20%. In another particular embodiment, the orodispersible tablet of the invention comprises croscarmellose sodium intragranular, in an amount from 1 to 35 % by weight of croscarmellose sodium in the orodispersible tablet, preferably from 5 to 25%, more preferably from 10 to 20%. In another particular embodiment, the orodispersible tablet of the invention comprises sodium starch glycolate intragranular, in an amount from 1 to 35 % by weight of sodium starch glycolate in the orodispersible tablet, preferably from 5 to 25%, more preferably from 10 to 20%.

In an embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix.

In a particular embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein rasagiline or a pharmaceutically acceptable salt thereof is trapped within the matrix, wherein the therapeutically effective amount of rasagiline or a pharmaceutically acceptable salt thereof per dosage form is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention comprises a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein the pharmaceutically acceptable polymer suitable for forming a matrix is an ion exchange resin, wherein rasagiline or a pharmaceutically acceptable salt thereof is trapped within the matrix, wherein the therapeutically effective amount of rasagiline or a pharmaceutically acceptable salt thereof per dosage form is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein the ion exchange resin is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers, wherein rasagiline or a pharmaceutically acceptable salt thereof is trapped within the matrix, wherein the therapeutically effective amount of rasagiline or a pharmaceutically acceptable salt thereof per dosage form is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein the ion exchange resin is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers, wherein rasagiline or a pharmaceutically acceptable salt thereof is trapped within the matrix, wherein the ion exchange resin is in a weight-to-weight ratio of rasagiline or pharmaceutically acceptable salt thereof to the ion exchange resin of from 1:1 to 1:10, wherein the therapeutically effective amount of rasagiline or a pharmaceutically acceptable salt thereof per dosage form is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein the ion exchange resin is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers, wherein at least 97% w/w of rasagiline or pharmaceutically acceptable salt thereof present in the orodispersible pharmaceutical solid dosage form is trapped within the matrix, wherein the ion exchange resin is in a weight-to-weight ratio of rasagiline or pharmaceutically acceptable salt thereof to the ion exchange resin of from 1:1 to 1:10, wherein the therapeutically effective amount of rasagiline or a pharmaceutically acceptable salt thereof per dosage form is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention is an orodispersible tablet which comprises a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein the ion exchange resin is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers, wherein at least 97% w/w of rasagiline or pharmaceutically acceptable salt thereof present in the orodispersible tablet is trapped within the matrix, wherein the ion exchange resin is in a weight-to-weight ratio of rasagiline or pharmaceutically acceptable salt thereof to the ion exchange resin of from 1:1 to 1:10, wherein the therapeutically effective amount of rasagiline or a pharmaceutically acceptable salt thereof per tablet is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention is an orodispersible tablet which comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein at least 97% w/w of rasagiline or pharmaceutically acceptable salt thereof present in the orodispersible tablet is trapped within the matrix, wherein polacrilin sodium is in a weight-to-weight ratio of rasagiline or pharmaceutically acceptable salt thereof to the polacrilin sodium of from 1:1 to 1:10, wherein the therapeutically effective amount of rasagiline or a pharmaceutically acceptable salt thereof per tablet is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base.

In a particular embodiment, the orodispersible pharmaceutical solid dosage form of the invention is an orodispersible tablet which comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein at least 97% w/w of rasagiline or pharmaceutically acceptable salt thereof present in the orodispersible tablet is trapped within the matrix, wherein polacrilin sodium is in a weight-to-weight ratio of rasagiline or pharmaceutically acceptable salt thereof to the polacrilin sodium of from 1:1 to 1:10, wherein the therapeutically effective amount of rasagiline or a pharmaceutically acceptable salt thereof per tablet is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base, and wherein the one or more pharmaceutically acceptable excipients comprise a diluent in an amount of 50 to 80% w/w relative to the total weight of the orodispersible tablet and a disintegrant in an amount of from 10% to 20% w/w relative to the total weight of the orodispersible tablet.

In a particular embodiment, the orodispersible pharmaceutical solid dosage form of the invention is an orodispersible tablet which comprises a therapeutically effective amount of rasagiline or a pharmaceutically acceptable salt thereof, polacrilin sodium suitable for forming a matrix with rasagiline or a pharmaceutically acceptable salt thereof, and one or more pharmaceutically acceptable excipients, wherein at least 97% w/w of rasagiline or pharmaceutically acceptable salt thereof present in the orodispersible tablet is trapped within the matrix, wherein polacrilin sodium is in a weight-to-weight ratio of rasagiline or pharmaceutically acceptable salt thereof to the polacrilin sodium of from 1:4 to 1:6, wherein the therapeutically effective amount of rasagiline or a pharmaceutically acceptable salt thereof per tablet is of from 0.05 mg to 5.0 mg, preferably of from 0.25 mg to 3.0 mg, more preferably of from 0.5 mg to 2.0 mg, and even more preferably of from 0.5 mg to 1.0 mg, calculated based on the weight of rasagiline free base, and wherein the one or more pharmaceutically acceptable excipients comprise mannitol in an amount of 50 to 80% w/w relative to the total weight of the orodispersible tablet and sodium croscarmellose in an amount of from 10% to 20% w/w relative to the total weight of the orodispersible tablet.

In an embodiment, an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein the dosage form is obtainable by the following process: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form.

In an embodiment, the orodispersible pharmaceutical solid dosage form of the invention is an orodispersible tablet comprising a therapeutically effective amount of rasagiline or a pharmaceutically acceptable salt thereof, an ion exchange resin suitable for forming a matrix with rasagiline or a pharmaceutically acceptable salt thereof, and one or more pharmaceutically acceptable excipients, wherein the tablet is obtainable by the following process: (i) dissolving rasagiline or a pharmaceutically acceptable salt thereof in water under acidic conditions, (ii) adding to the solution of step (i) an ion exchange resin suitable for forming a matrix with rasagiline or a pharmaceutically acceptable salt thereof and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible tablet.

In an embodiment, the orodispersible pharmaceutical solid dosage form of the invention is an orodispersible tablet comprising a therapeutically effective amount of rasagiline or a pharmaceutically acceptable salt thereof, sulfonated styrene-divinylbenzene copolymer suitable for forming a matrix with rasagiline or a pharmaceutically acceptable salt thereof, and wherein the sulfonated styrene-divinylbenzene copolymer preferably is polacrilin sodium, and one or more pharmaceutically acceptable excipients, wherein the tablet is obtainable by the following process: (i) dissolving rasagiline or a pharmaceutically acceptable salt thereof in water under acidic conditions, (ii) adding to the solution of step (i) sulfonated styrene-divinylbenzene copolymer suitable for forming a matrix with rasagiline or a pharmaceutically acceptable salt thereof, and wherein the sulfonated styrene-divinylbenzene copolymer preferably is polacrilin sodium, and stirring the obtained suspension for at least 2 hours; (iii) separately providing mannitol and optionally peppermint, menthol and potassium acesulfame, or other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with sodium stearyl fumarate and optionally peppermint, menthol and potassium acesulfame, or other excipients; (vii) compressing the mixture obtained in step (vi) to form an orodispersible tablet.

In an embodiment, an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein the dosage form is obtainable by the following process: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form; and wherein said dosage form exhibits a dissolution profile according to which (a) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (b) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (c) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute.

In an embodiment, an orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients, wherein the dosage form is obtainable by the following process: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form; and wherein said dosage form exhibits a dissolution profile according to which (a) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (b) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (c) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

In an embodiment, the orodispersible pharmaceutical solid dosage form of the invention is an orodispersible tablet comprising a therapeutically effective amount of rasagiline or a pharmaceutically acceptable salt thereof, sulfonated styrene-divinylbenzene copolymer suitable for forming a matrix with rasagiline or a pharmaceutically acceptable salt thereof, and wherein the sulfonated styrene-divinylbenzene copolymer preferably is polacrilin sodium, and one or more pharmaceutically acceptable excipients, wherein the tablet is obtainable by the following process: (i) dissolving rasagiline or a pharmaceutically acceptable salt thereof in water under acidic conditions, (ii) adding to the solution of step (i) sulfonated styrene-divinylbenzene copolymer suitable for forming a matrix with rasagiline or a pharmaceutically acceptable salt thereof, and wherein the sulfonated styrene-divinylbenzene copolymer preferably is polacrilin sodium, and stirring the obtained suspension for at least 2 hours; (iii) separately providing mannitol and optionally peppermint, menthol and potassium acesulfame, or other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with sodium stearyl fumarate and optionally peppermint, menthol and potassium acesulfame, or other excipients; (vii) compressing the mixture obtained in step (vi) to form an orodispersible tablet; and wherein said dosage form exhibits a dissolution profile according to which (a) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and (b) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and (c) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute.

In an embodiment, the process for the preparation of the orodispersible pharmaceutical dosage form of the invention comprises: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form.

In an embodiment, the process for the preparation of the orodispersible pharmaceutical dosage form of the invention comprises: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii), as a direct granulating solvent to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible pharmaceutical solid dosage form.

In an embodiment, the process for the preparation of the orodispersible pharmaceutical dosage form of the invention provides an orodispersible tablet, wherein the process comprises: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible tablet.

In an embodiment, the process for the preparation of the orodispersible pharmaceutical dosage form of the invention provides an orodispersible tablet, wherein the process comprises: (i) dissolving rasagiline or a pharmaceutically acceptable salt thereof in water under acidic conditions, (ii) adding to the solution of step (i) a pharmaceutically acceptable polymer suitable for forming a matrix with rasagiline or a pharmaceutically acceptable salt thereof, and stirring the obtained suspension for at least 2 hours; (iii) separately providing one or more diluents or a mixture thereof with one or more of disintegrants, sweeteners, flavouring agent, and other excipients; (iv) wet granulating either the one or more diluents or the mixture each provided in step (iii) with the suspension of step (ii) as a direct granulating solvent to obtain wet granules; (v) drying the wet granules obtained in step (iv) to obtain dry granules; (vi) mixing the dry granules obtained in step (v) with one or more lubricants and optionally with one or more of diluents, disintegrants, sweeteners, flavouring agents, and other excipients; (vii) compressing the mixture obtained in step (vi) to form an orodispersible tablet.

In a preferred embodiment, the pharmaceutically acceptable polymer suitable for forming a matrix of step (i) is an ion exchange resin, preferably a cation exchange resin, more preferably an acidic or weakly acidic cation exchange resin. In another embodiment, pharmaceutically acceptable polymer suitable for forming a matrix of step (i) is a partial potassium salt of a methacrylic acid-divinylbenzene copolymer or a partial sodium salt of a sulfonated styrene-divinylbenzene copolymer. In a preferred embodiment, the one or more disintegrants of steps (iii) and (vi) are selected from the group of croscarmellose sodium, crospovidone and sodium starch glycolate.

Drying step (v) of the process of the invention may be performed by any method known in the state of the art for drying granules in a wet granulation process. The drying temperature and the duration of drying process depend on the nature of the active ingredient and the level of moisture required for the successful production of satisfactory tablets. Generally, shelf or tray drier and fluidized-bed drier can be used for this purpose. In an embodiment, drying step (v) of the process of the invention is performed under vacuum conditions, preferably from 50 to 70°C, more preferably from 55 to 65°C. Generally, the term "vacuum conditions" refers to a pressure from 0.5 mbar to 3 mbar; preferably comprised from 1 to 2 mbar.

In an preferred embodiment, the process for the preparation of the orodispersible pharmaceutical dosage form of the invention provides an orodispersible tablet, wherein the process comprises: (i) dissolving rasagiline or a pharmaceutically acceptable salt thereof in water under acidic conditions, (ii) adding to the solution of step (i) sulfonated styrene-divinylbenzene copolymer suitable for forming a matrix with rasagiline or a pharmaceutically acceptable salt thereof, and wherein the sulfonated styrene-divinylbenzene copolymer preferably is polacrilin sodium, and stirring the obtained suspension for at least 2 hours; (iii) separately providing mannitol and optionally peppermint, menthol and potassium acesulfame, or other excipients; (iv) wet granulating either the one or more diluents or the mixture each provided in step (iii) with the suspension of step (ii) to obtain wet granules; (v) drying the wet granules obtained in step (iv) to obtain dry granules; (vi) mixing the dry granules obtained in step (v) with sodium stearyl fumarate and optionally peppermint, menthol and potassium acesulfame, or other excipients; (vii) compressing the mixture obtained in step (vi) to form an orodispersible tablet.

All the embodiments disclosed above for the orodispersible pharmaceutical solid dosage form of the invention apply also for the preparation process.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Example 1: Manufacturing process of orodispersible tablets of the invention

Rasagiline tartrate is dissolved in water under at pH=2. Once it is entirely dissolved, polacrilin resin C100NaMR is added to the solution, and the suspension is stirred for at least 2 hours. The resulting suspension, which contains the rasagiline trapped within the matrix formed with polacrilin resin C100NaMR, is used directly as granulating solvent of a mixture of mannitol, sodium croscarmellose and hydroxypropylmethyl cellulose. Said granulation and subsequent drying process are carried out in a shear/mixer with drying capabilities. Alternatively, the drying process may be carried out in a fluid bed dryer or equivalent equipment. The obtained dried granules are then sieved through 1 mm. The resulting granules are weighed, and the yield is obtained in order to recalculate the necessary amount of extragranular excipients, such as peppermint, menthol and potassium acesulfame. Said extragranular excipients are sieved through 1 mm and then mixed with the granules for at least 10 minutes at 15 rpm. Sodium stearyl fumarate is sieved through 1 mm and then mixed for at least 5 minutes at 15 rpm. The final mixture is then compressed using 8 mm punches.

Orodispersible tablets of Tables 1-4 were similarly prepared according to the manufacturing process described in the paragraph before. Alternatively, first step of the manufacturing process, i.e. the dissolution of rasagiline tartrate and formation of a matrix with a pharmaceutically acceptable polymer, wherein rasagiline is trapped within such formed matrix, may be carried out in organic solvents or mixtures of water and organic solvents.

Further orodispersible tablets were also prepared comprising rasagiline mesylate as pharmaceutically acceptable salt of rasagiline.

**Table 1: Composition of formulations 1 and 2.**

| | **Formulation 1** | | | **Formulation 2** | | |
|---|---|---|---|---|---|---|
| | Intragr. (mg) | Extragr. (mg) | % total | Intragr. (mg) | Extragr. (mg) | % total |
| Rasagiline tartrate | 1.44 | - | 0.96 | 1.44 | - | 0.96 |
| Mannitol | 110.55 | - | 73.70 | 107.67 | - | 71.78 |
| Peppermint flavour | - | 1.50 | 1.00 | - | 1.50 | 1.00 |
| Menthol flavour | - | 1.50 | 1.00 | - | 1.50 | 1.00 |
| Sodium croscarmellose | 22.50 | - | 15.00 | 22.50 | - | 15.00 |
| Hydroxypropyl methyl cellulose | 3.00 | - | 2.00 | 3.00 | - | 2.00 |
| Acesulfame K | - | 3.00 | 2.00 | - | 3.00 | 2.00 |
| Resin IR-120 | 4.31 | - | 2.88 | 7.19 | - | 4.79 |
| Sodium stearyl fumarate | - | 2.20 | 1.47 | - | 2.20 | 1.47 |
| Total Intra/Extra (mg) | 141.80 | 8.20 | - | 107.80 | 42.20 | - |
| Total core (mg) | 150.00 | | - | 150.00 | | - |

**Table 2: Composition of formulations 3 and 4.**

| | **Formulation 3** | | | **Formulation 4** | | |
|---|---|---|---|---|---|---|
| | Intragr. (mg) | Extragr. (mg) | % total | Intragr. (mg) | Extragr. (mg) | % total |
| Rasagiline tartrate | 1.44 | - | 0.96 | 1.44 | - | 0.96 |
| Mannitol | 76.23 | - | 50.82 | 42.23 | 34.00 | 50.82 |
| Polacrilin resin C100NaMR | 8.63 | - | 5.75 | 8.63 | - | 5.75 |
| Peppermint flavour | - | 1.50 | 1.00 | - | 1.50 | 1.00 |
| Menthol flavour | - | 1.50 | 1.00 | - | 1.50 | 1.00 |
| Lactose monohydrate | 30.71 | - | 20.47 | 30.71 | - | 20.47 |
| Sodium croscarmellose | 22.50 | - | 15.00 | 22.50 | - | 15.00 |
| Hydroxypropyl methyl cellulose | 3.00 | - | 2.00 | 3.00 | - | 2.00 |
| Acesulfame K | - | 3.00 | 2.00 | - | 3.00 | 2.00 |
| Magnesium stearate | - | 1.50 | 1.00 | - | 1.50 | 1.00 |
| Total Intra/Extra (mg) | 142.50 | 7.50 | - | 108.50 | 41.50 | - |
| Total core (mg) | 150.00 | | - | 150.00 | | - |

**Table 3: Composition of formulations 5 and 6.**

| | **Formulation 5** | | | **Formulation 6** | | |
|---|---|---|---|---|---|---|
| | Intragr. (mg) | Extragr. (mg) | % total | Intragr. (mg) | Extragr. (mg) | % total |
| Rasagiline tartrate | 1.44 | - | 0.96 | 1.44 | - | 0.96 |
| Mannitol | 109.11 | - | 72.74 | 107.67 | - | 71.78 |
| Peppermint flavour | - | 1.50 | 1.00 | - | 1.50 | 1.00 |
| Menthol flavour | - | 1.50 | 1.00 | - | 1.50 | 1.00 |
| Sodium croscarmellose | 22.50 | - | 15.00 | 22.50 | - | 15.00 |
| Hydroxypropyl methyl cellulose | 3.00 | - | 2.00 | 3.00 | - | 2.00 |
| Acesulfame K | - | 3.00 | 2.00 | - | 3.00 | 2.00 |
| Polacrilin resin C100NaMR | 5.75 | - | 3.84 | 7.19 | - | 4.79 |
| Sodium stearyl fumarate | - | 2.20 | 1.47 | - | 2.20 | 1.47 |
| Total Intra/Extra (mg) | 141.80 | 8.20 | - | 141.80 | 8.20 | - |
| Total core (mg) | 150.00 | | - | 150.00 | | - |

**Table 4: Composition of formulations 7 and 8.**

| | **Formulation 7** | | | **Formulation 8** | | |
|---|---|---|---|---|---|---|
| | Intragr. (mg) | Extragr. (mg) | % total | Intragr. (mg) | Extragr. (mg) | % total |
| Rasagiline tartrate | 1.44 | - | 0.96 | 1.44 | - | 0.96 |
| Mannitol | 106.23 | - | 70.82 | 42.23 | 34.00 | 50.82 |
| Peppermint flavour | - | 1.50 | 1.00 | - | 1.50 | 1.00 |
| Menthol flavour | - | 1.50 | 1.00 | - | 1.50 | 1.00 |
| Lactose monohydrate | - | - | - | 30.71 | - | 20.47 |
| Sodium croscarmellose | 22.50 | - | 15.00 | 22.50 | - | 15.00 |
| Hydroxypropyl methyl cellulose | 3.00 | - | 2.00 | 3.00 | - | 2.00 |
| Acesulfame K | - | 3.00 | 2.00 | - | 3.00 | 2.00 |
| Polacrilin resin C100NaMR | 8.63 | - | 5.75 | - | - | - |
| Polacrilin resin C115KMR | - | - | - | 8.63 | - | 5.75 |
| Sodium stearyl fumarate | - | 2.20 | 1.47 | - | - | - |
| Magnesium stearate | - | - | - | - | 1.50 | 1.00 |
| Total Intra/Extra (mg) | 141.80 | 8.20 | - | 108.50 | 41.50 | - |
| Total core (mg) | 150.00 | | - | 150.00 | | - |

### Example 2: Friability test and disintegration test of orodispersible tablets of the invention

Formulations 1 to 8, as described in Tables 1 to 4, were obtained following the process described in Example 1. Said Formulations were submitted for friability test in accordance with the guidelines of the European Pharmacopeia, edition 10.0, pp. 336-337. The results are shown in Table 5. All tablets were compressed using a punch of 8 mm.

Said Formulations 1 to 8 were also tested for disintegration time following Test A of the European Pharmacopeia, edition 10.0, page 323), water having pH=7, at 37°C and 30 cycles per minute. The results are also shown in Table 5.

**Table 5: Friability results and disintegration time of Formulations 1 to 8 of Tables 1 to 4.**

| **Tablet** | Hardness (N) | Thickness (mm) | Disintegration time (s) | Friability (%) |
|---|---|---|---|---|
| **Formulation 1** | 42 | 3.10 | 44 | 0.12 |
| **Formulation 2** | 30 | 3.15 | 28 | 0.81 |
| **Formulation 3** | 26 | 3.07 | 24 | 0.37 |
| **Formulation 4** | 19 | 3.00 | 22 | 0.7 |
| **Formulation 5** | 28 | 3.16 | 28 | 0.22 |
| **Formulation 6** | 27 | 3.19 | 21 | 0.24 |
| **Formulation 7** | 49 | 2.96 | 28 | 0.06 |
| **Formulation 8** | 17 | 3.10 | 28 | 0.42 |

As shown in Table 5, all orodispersible tablets of Formulations 1 to 8 complied with the requirement of having a friability of less than 1 % in accordance with guidelines of the European Pharmacopeia. Moreover, all orodispersible tablets of Formulations 1 to 8 complied with the requirement of being orodispersible tablets, in accordance with the definition given by the European Pharmacopeia, edition 10.0, page 939, wherein orodispersible tablets are defined as non-coated tablets for placing in the mouth which disintegrate quickly before they are swallowed. It also establishes 3 minutes as the time under which they must disintegrate in the disintegration test for tablets and capsules, according to the disintegration Test A of the European Pharmacopeia, edition 10.0, page 323, water having pH=7, at 37°C and 30 cycles per minute.

### Example 3: Dissolution profiles of the formulations of the invention at buccal pH.

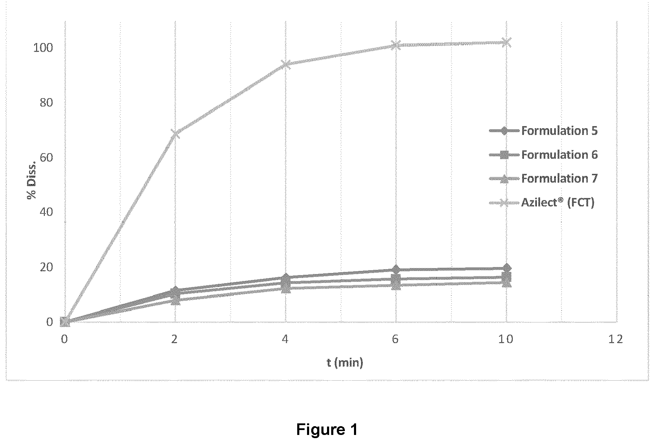

Dissolution profiles of Formulations 5, 6 and 7, as obtained in Example 1, and a commercial film coated tablet (FCT) of Azilect^{®} (1.0 mg strength) were measured at buccal pH. The results are shown in Table 6 and Figure 1.

**Table 6: Dissolution profiles of Azilect^{®} and Formulations 5, 6 and 7 at buccal pH**

| | **Azilect^{®} (FCT)** | | **Formulation 5** | | **Formulation 6** | | **Formulation 7** | |
|---|---|---|---|---|---|---|---|---|
| T (min) | Dis. (%) | CV (%) | Dis. (%) | CV (%) | Dis. (%) | CV (%) | Dis. (%) | CV (%) |
| 0 | 0.0 | 0.0 | 0.0 | 0.0 | 0.0 | 0.0 | 0.0 | 0.0 |
| 2 | 68.6 | 19.7 | 11.4 | 9.2 | 10.3 | 8.4 | 7.9 | 11.3 |
| 4 | 93.9 | 9.8 | 16.2 | 6.5 | 14.3 | 5.1 | 12.2 | 4.0 |
| 6 | 101.0 | 2.9 | 19.1 | 5.4 | 15.7 | 6.7 | 13.4 | 3.1 |
| 10 | 102.1 | 2.5 | 19.7 | 6.1 | 16.4 | 6.4 | 14.5 | 3.5 |

**Table 7: Experimental conditions of the dissolution profiles at buccal pH**

| **Samples** | **Azilect^{®} (FCT)** | **Formulations 5, 6, 7** |
|---|---|---|
| pH | pH=7 | pH=7 |
| Temperature | 37 °C | 37 °C |
| Volume | 500 mL | 500 mL |
| Revolutions per minute | 50 | 50 |
| Eur. Ph. Apparatus | Apparatus II (paddles) | Apparatus II (paddles) |
| N | 6 | 6 |

The dissolution profiles show that the dosage forms of the invention have a very low release of rasagiline at buccal pH, much lower than Azilect^{®} under the same conditions.

### Example 4: Dissolution profiles of the formulations of the invention at gastric pH.

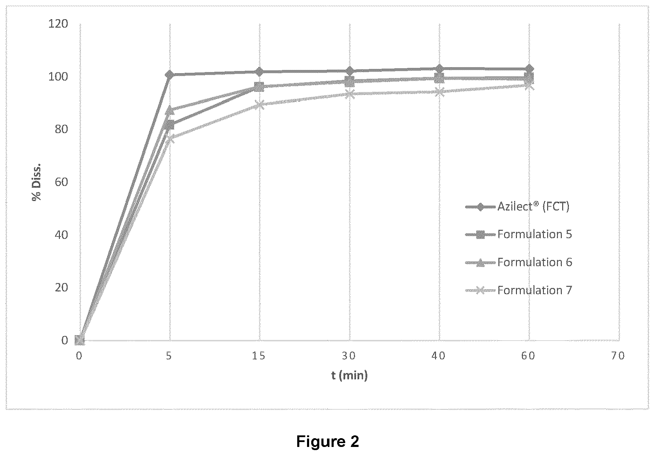

Dissolution profiles of Formulations 5, 6 and 7, as obtained in Example 1, and a commercial film coated tablet (FCT) of Azilect^{®} (1.0 mg strength) were measured at gastric pH. The results are shown in Table 8 and Figure 2.

**Table 8: Dissolution profiles of Azilect^{®} and Formulations 5, 6 and 7 at gastric pH**

| | **Azilect^{®} (FCT)** | | **Formulation 5** | | **Formulation 6** | | **Formulation 7** | |
|---|---|---|---|---|---|---|---|---|
| T (min) | Dis. (%) | CV (%) | Dis. (%) | CV (%) | Dis. (%) | CV (%) | Dis. (%) | CV (%) |
| 0 | 0,0 | 0,0 | 0.0 | 0.0 | 0.0 | 0.0 | 0,0 | 0,0 |
| 5 | 100,6 | 6,2 | 81.7 | 6.9 | 87.4 | 5.4 | 76,6 | 2,7 |
| 15 | 101,8 | 1,7 | 96.0 | 1.4 | 96.2 | 2.4 | 89,4 | 1,3 |
| 30 | 102,1 | 2,0 | 98.3 | 1.1 | 97.9 | 1.7 | 93,4 | 1,0 |
| 40 | 103,0 | 0,6 | 99.4 | 1.8 | 99.3 | 2.5 | 94,2 | 0,9 |
| 60 | 102,9 | 0,7 | 99.6 | 0.7 | 99.0 | 1.9 | 96,8 | 1,0 |

**Table 9: Experimental conditions of the dissolution profiles at gastric pH**

| **Samples** | **Azilect^{®} (FCT)** | **Formulations 5, 6, 7** |
|---|---|---|
| pH | pH=1.2 | pH=1.2 |
| Temperature | 37 °C | 37 °C |
| Volume | 50 mL | 50 mL |
| Revolutions per minute | 100 | 100 |
| Eur. Ph. Apparatus | Orbital bath | Orbital bath |
| N | 6 | 6 |

### Example 5: Stability of formulations of the invention

Formulations as obtained in Example 1 were analyzed to quantify the amount of indan-1-amine impurity (also known as "impurity I" of rasagiline) present after being stored for a certain amount of time under controlled conditions of temperature and Relative Humidity (RH). The results are shown in Table 10.

**Table 10: Weight percentage of indan-1-amine (also known as 1-Aminoindan or "impurity I" of rasagiline) present in the dosage forms of the invention over time and the amount of total impurities.**

| | | **indan-1-amine (% w/w)** | | |
|---|---|---|---|---|
| **Storage conditions T (°C) RH (%)** | **Time (months)** | **Azilect^{®} (FCT)** | **Formulation 6** | **Formulation 7** |
| 25 °C 60% RH | 0 | 0.05 | ND | ND |
| | 1 | N/A | ND | ND |
| | 3 | 0.12 | N/A | N/A |
| 40 °C 75% RH | 0 | 0.05 | ND | <0.05 |
| | 1 | N/A | ND | <0.05 |
| | 3 | 0.40 | N/A | 0.24 |

| | | | | |
|---|---|---|---|---|
| N/A stands for not available. ND stands for not detected. | | | | |

In all the cases the content of indan-1-amine impurity was well below the qualification threshold (0.5%) that ICH establishes for degradation products in its Guidelines for Impurities in New Drug Products Q3B(R2).

### Example 6: Percentage of the active ingredient trapped within the matrix formed with the ion exchange resin

The percentage of the active ingredient trapped within the matrix formed with the ion exchange resin was analysed. Rasagiline tartrate was stirred in water and the pH was lowered until pH=2 by adding HCI solution. The ion exchange resin was added to the solution and stirring was maintained for 3 hours. At that time (T1), a sample of 5 mL is taken, and it is filtered. The liquid phase obtained from the filtration is analysed by HPLC to quantify the amount of rasagiline; i.e. rasagiline not trapped within the matrix formed with the ion exchange resin. The corresponding percentage of rasagiline trapped within the matrix formed at T1 is calculated by difference between total amount of rasagiline minus amount of rasagiline not trapped within the matrix.

At time 6 hours (T2), another sample of 5 mL is taken and filtered. The same procedure as the one done at time T1 is repeated. Results are shown in Table 11.

**Table 11: % of rasagiline trapped within the matrix formed with the ion exchange resin**

| Resin | API:resin ratio | % rasagiline not trapped within the matrix formed | % rasagiline trapped within the matrix formed |
|---|---|---|---|
| Polacrilin resin C100NaMR | 1:4 | T1: 2.8% | T1: 97.2% |
| | | T2: 2.7% | T2: 97.3% |
| Polacrilin resin C100NaMR | 1:5 | T1: 2.1% | T1: 97.9% |
| | | T2: 2.1% | T2: 97.9% |

### Example 7: Bioavailability test

First studies show that the dissolution behavior defined by the claimed dissolution and disintegration profiles allows the orodispersible formulation of the invention to provide for a bioavailability sufficiently equivalent to the one of the conventional film coated immediate release tablets of the prior art (i.e. Azilect^{®}).

### Citation List

European Pharmacopeia, edition 10.0, page 939
European Pharmacopeia, edition 10.0, page 323
European Pharmacopeia, edition 10.0, pp. 336-337
ICH Guidelines for Impurities in New Drug Products Q3B(R2)
Comoglu et al. (Formulation, in vitro and in vivo evaluation of taste masked rasagiline orally fast disintegrating tablets (ODTS), Research & Reviews in Pharmacy and Pharmaceutical Sciences, 2017, vol. 6(2): 27-38)
Tabi et al., (The pharmacokinetic evaluation of selegiline ODT for the treatment of Parkinson's disease, Expert Opin. Drug Metab. Toxicol., 2013; vol. 9(5): 629-36)
WO2012015946
WO2006057912
WO2013168032
Dimpfel, W. et al., Effects of rasagiline, its metabolite aminoindan and selegiline on glutamate receptor mediated signaling in the rat hippocampus slice in vitro, BMC Pharmacol., 2011, 11:2

## Claims

1. An orodispersible pharmaceutical solid dosage form comprising a therapeutically effective amount of rasagiline or a pharmaceutically acceptable salt thereof, a pharmaceutically acceptable polymer suitable for forming a matrix with rasagiline or a pharmaceutically acceptable salt thereof, and one or more pharmaceutically acceptable excipients,
wherein rasagiline or a pharmaceutically acceptable salt thereof is trapped within the matrix, and wherein said dosage form exhibits a dissolution profile according to which
(i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and
(ii) after 15 minutes at pH=1.2, more than 75% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and
(iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and
wherein said orodispersible pharmaceutical solid dosage form is disintegrated in less than 3 minutes, wherein disintegration test was performed using a European Pharmacopeia disintegration apparatus A, placing the dosage form in water having pH=7 at 37 °C and 30 cycles per minute.

2. The orodispersible pharmaceutical solid dosage form according to claim 1, wherein the pharmaceutically acceptable polymer suitable for forming a matrix with rasagiline or a pharmaceutically acceptable salt thereof is an ion exchange resin.

3. The orodispersible pharmaceutical solid dosage form according to any of the claims 1-2, wherein the solid dosage form is an orodispersible tablet.

4. The orodispersible pharmaceutical solid dosage form according to any of the claims 1-3, wherein the therapeutically effective amount of rasagiline or a pharmaceutically acceptable salt thereof per dosage form is of from 0.05 to 5.0 mg, calculated based on the weight of rasagiline free base.

5. The orodispersible pharmaceutical solid dosage form according to any of the claims 2-4, wherein the ion exchange resin is in a weight-to-weight ratio of rasagiline or pharmaceutically acceptable salt thereof to the ion exchange resin of from 1:1 to 1:10.

6. The orodispersible pharmaceutical solid dosage form according to any of the claims 2-5, wherein the ion exchange resin is selected from the group of sodium or potassium or magnesium or calcium salts or partial sodium or potassium or magnesium or calcium salts of sulfonated polystyrene polymers, sulfonated styrene-divinylbenzene copolymers, polymethacrylic acid polymers, methacrylic acid-divinylbenzene copolymers, protonated sulfonated polystyrene polymers, protonated sulfonated styrene-divinylbenzene copolymers, protonated polymethacrylic acid polymers and protonated methacrylic acid-divinylbenzene copolymers.

7. The orodispersible pharmaceutical solid dosage form according to claim 6, wherein the ion exchange resin is a sulfonated styrene-divinylbenzene copolymer, and wherein the sulfonated styrene-divinylbenzene copolymer preferably is polacrilin sodium.

8. The orodispersible pharmaceutical solid dosage form according to any of the claims 1-7, wherein at least 97% w/w of rasagiline or pharmaceutically acceptable salt thereof present in the orodispersible pharmaceutical solid dosage form is trapped within the matrix formed with the pharmaceutically acceptable polymer.

9. The orodispersible pharmaceutical solid dosage form according to any of claims 1-8, wherein said dosage form comprises one or more disintegrants in an amount of from 1% to 30% w/w relative to the total weight of the dosage form.

10. The orodispersible pharmaceutical solid dosage form according to any of claims 1-9, wherein said pharmaceutically acceptable excipient is one or more intragranular disintegrants.

11. The orodispersible pharmaceutical solid dosage form according to any of claims 1-10, wherein the pharmaceutically acceptable salt of rasagiline is rasagiline tartrate or rasagiline mesylate.

12. The orodispersible pharmaceutical solid dosage form according to any of claims 1-11, wherein the dosage form exhibits a dissolution profile according to which
(i) after 2 minutes at pH=7.0, less than 12% w/w of the rasagiline relative to the total rasagiline content of the dosage form is dissolved, wherein the amount of dissolved rasagiline is determined using an Apparatus II (paddles), placing the dosage form in 500 mL, under pH=7.0, at 37 °C and stirring at 50 revolutions per minute, and
(ii) after 15 minutes at pH=1.2, more than 85% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute, and
(iii) after 40 minutes at pH=1.2, at least 90% w/w of the rasagiline relative to the total rasagiline content of the dosage form is dissolved, wherein the amount of dissolved rasagiline is determined using an orbital bath, placing the dosage form in 50 mL, under pH=1.2, at 37 °C and stirring at 100 revolutions per minute.

13. The orodispersible pharmaceutical solid dosage form according to any of claims 1-12, wherein said orodispersible pharmaceutical solid dosage form is disintegrated in less than 2 minutes, particularly in less than 1 minute, and more particularly in less than 45 seconds, wherein disintegration test was performed using a European Pharmacopeia disintegration apparatus A, placing the dosage form in water having pH=7 at 37 °C and 30 cycles per minute.

14. A process for the preparation of the orodispersible pharmaceutical solid dosage form as defined according to any of claims 1-13, which comprises:
(i) dissolving rasagiline or a pharmaceutically acceptable salt thereof in water under acidic conditions,
(ii) adding to the solution of step (i) a pharmaceutically acceptable polymer suitable for forming a matrix with rasagiline or a pharmaceutically acceptable salt thereof, and stirring the obtained suspension for at least 2 hours;
(iii) separately providing one or more diluents or a mixture thereof with one or more of disintegrants, sweeteners, flavouring agent, and other excipients;
(iv) wet granulating either the one or more diluents or the mixture each provided in step (iii) with the suspension of step (ii) to obtain wet granules;
(v) drying the wet granules obtained in step (iv) to obtain dry granules;
(vi) mixing the dry granules obtained in step (v) with one or more lubricants and optionally with one or more of diluents, disintegrants, sweeteners, flavouring agents, and other excipients;
(vii) compressing the mixture obtained in step (vi) to form an orodispersible pharmaceutical solid dosage form.

15. An orodispersible pharmaceutical solid dosage form according to any of claims 1-13 for use in the treatment of idiopathic Parkinson's disease as monotherapy (without levodopa) or as adjunct therapy (with levodopa) in patients with end of dose fluctuations.
